# EUROPEAN PATENT APPLICATION

(11) **EP 4 205 753 A1**
(43) Date of publication of application: **05.07.2023**
(21) Application number: 21306961.0
(22) Date of filing: 30.12.2021
(51) Int. Cl.: A61K 36/47, A61K 36/73, A61K 36/9062, A61K 36/06, A61P 31/12

(54) **ANTIVIRAL PLANT EXTRACT COMPOSITION AND METHOD FOR PREPARING SUCH COMPOSITION**

(71) Applicant: Cariel, Léon, 75007 Paris (FR)
(72) Inventor: Cariel, Léon, 75007 Paris (FR)
(74) Representative: Icosa

(57) **Abstract**

The present invention relates to a herbal composition comprising a first composition comprising an extract of *Alpinia galanga* and a second composition comprising an extract of *Phyllanthus emblica,* wherein the first and/or the second compositions are fermented compositions with at least one yeast. The invention further relates to a process for preparing such a herbal composition. Lastly, the invention relates to the uses of the herbal composition.

## Description

### FIELD OF INVENTION

The present invention relates to a herbal composition comprising a mixture of plant extracts. The invention further relates to a process for obtaining said composition as well as to its pharmaceutical use.

### BACKGROUND OF INVENTION

The outbreak of coronavirus disease 2019 (COVID-19) was reported by Tang and colleagues late December 2019 in Wuhan, China, with a series of respiratory infections caused by a novel coronavirus, severe acute respiratory syndrome coronavirus 2 (Sars-CoV-2). By March 2020, 105,586 cases of COVID-19 pneumonia were laboratory-confirmed in over 100 countries worldwide. By March 2021, more than 2.5 million deaths were caused by COVID-19 worldwide. With deeper understanding of the biological characteristic of Sars-CoV-2, a progress has been made in COVID-19 treatment. For example, the administration of remdesivir has been reported as a possible treatment against COVID-19.

However, considering remdesivir's elevated cost and the broad spectrum of side effects, there is a substantial need to supply further efficient antiviral treatments with an improved safety profile and that do not need to be prepared with high-cost synthetic means.

Plant extracts have been proposed in the art in order to address the above shortcomings. However, their administration is often hindered by bioavailability and solubility obstacles.

The Applicant has surprisingly found that a herbal composition, typically a fermented herbal composition, as hereinafter described, can exert an antiviral effect and overcome the bioavailability and solubility obstacles.

### SUMMARY

According to a first aspect, the invention relates to a herbal composition comprising a first composition comprising an extract of *Alpinia galanga* and a second composition comprising an extract of *Phyllanthus emblica,* wherein the first and/or the second compositions are fermented compositions with at least one yeast.

In one embodiment, the first and the second compositions are fermented compositions that have been previously mixed and jointly fermented with at least one fungal strain. Typically, the at least one fungal strain is *Saccharomyces cerevisiae.*

According to a typical embodiment, the first composition consists of an *Alpinia galanga* rhizome extract and/or the second composition consists of an extract of *Phyllanthus emblica* fruit extract. Typically, the amount of the first and/or the second composition ranges from 5 % to 60 % in weight relative to the total weight of the composition.

Optionally, the composition may further comprise a third composition comprising *Filipendula ulmaria* or an extract thereof.

In one embodiment, the herbal composition, typically the fermented herbal composition, presents an aqueous solubility of more than 5 mg/mL at 25°C.

The invention further relates to a pharmaceutical composition comprising the composition according as described above and at least one pharmaceutically acceptable excipient.

The invention further relates to a nutraceutical composition comprising the composition as described above and at least one nutraceutically acceptable excipient.

According to a further aspect, the invention relates to the composition or the pharmaceutical composition according to the invention, for use as a drug.

According to a yet further aspect, the invention relates to the composition or the pharmaceutical composition according to the invention for use in the prevention and/or the treatment of a viral infection in a subject in need thereof.

In one embodiment, the viral infection is a pulmonary viral infection by at least one peplomer (spike) bearing virus selected from the group consisting of orthomyxoviruses, paramyxoviruses, rhabdoviruses, filoviruses, coronaviruses, bunyaviruses, arenaviruses, and retroviruses, preferably from coronaviruses.

In one typical embodiment, the viral infection is an infection by the virus SARS-CoV-2.

Typically, the composition is orally administered to the subject at least once a day.

Lastly, the invention relates to a process for preparing a composition according to the invention, comprising the steps of:
i. supplying a first composition comprising or consisting of an extract of *Alpinia galanga,* preferably an extract of *Alpinia galanga* rhizomes,
ii. supplying a second composition comprising an extract of *Phyllanthus emblica,* preferably an extract of *Phyllanthus emblica* fruits,
iii. optionally supplying a third composition comprising or consisting of *Filipendula ulmaria* or an extract thereof,
iv. mixing the first with the second compositions and optionally with the third composition, leading to a herbal composition,
v. fermenting the herbal composition with at least one yeast, preferably at least one Saccharomycetaceae strain, even more preferably *Saccharomyces cerevisiae,* leading to a fermented herbal composition,
vi. optionally condensing the fermented herbal composition, leading to a condensed herbal composition, and
vii. optionally mixing the fermented herbal composition of step (v) or the condensed herbal composition of step (vi) with at least one pharmaceutically and/or nutraceutically acceptable excipient.

### DEFINITIONS

In the present invention, the following terms have the following meanings:

**"About"** preceding a figure means plus or less 10% of the value of said figure, preferably plus or less 5% of the value of said figure, in particular plus or less 1% of the value of said figure.

**"Excipient"** refers to any inactive ingredient which is required for the formulation of an active agent in a suitable dosage form. Excipients are materials suitable for administration and include any such material known in the art which is non-toxic and which does not interact with any components of the composition in a deleterious manner. In one embodiment, "Excipients" refers to any and all solvents, diluents, carriers, fillers, bulking agents, binders, disintegrants, polymer, lubricant, glidant, surfactants, isotonic agents, thickening or emulsifying agents, stabilizers, absorption accelerators, flavoring agents, preservatives, antioxidants, buffering agents, or any combination thereof.

**"Pharmaceutical composition"** refers to a composition comprising an active principle in association with a pharmaceutically acceptable vehicle or excipient. A pharmaceutical composition is for therapeutic use, and relates to health. Especially, a pharmaceutical composition may be indicated for treating a disease or disorder.

By **"pharmaceutically acceptable"** is meant that the ingredients of a pharmaceutical composition are compatible with each other and not deleterious to the subject to which/whom it is administered.

**"Pharmaceutically acceptable excipient"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA. The terms **"pharmaceutically acceptable excipient", "pharmaceutically acceptable carrier"** or **"pharmaceutical vehicle"** refer to an inert medium or carrier used as a solvent or diluent in which the pharmaceutically active ingredient is formulated and/or administered, and which does not produce an adverse, allergic or other reaction when administered to an animal, preferably a human being. For the purposes of the invention, **"pharmaceutically acceptable excipient"** includes all pharmaceutically acceptable excipients as well as all pharmaceutically acceptable carriers, diluents, and/or adjuvants.

The term **"therapeutically effective amount"** (or more simply **"effective amount")** refers to the amount of active agent, herein the herbal composition, alone or as part of a pharmaceutical composition, that is aimed at, without causing significant negative or adverse side effects to the subject in need of treatment, preventing, reducing, alleviating or slowing down (lessening) one or more of the symptoms of a condition, symptom, disorder or disease. **"Pharmaceutically effective amount"** refers to the amount of a pharmaceutical composition necessary and sufficient for slowing down or stopping the progression, aggravation, or deterioration of one or more symptoms of a disease, disorder or condition; or alleviating the symptoms of a disease, disorder or condition; or curing the disease, disorder or condition.

**"Nutraceutical composition"** refers to a composition comprising an edible ingredient allegedly providing a physiological benefit. A nutraceutical composition is for non-therapeutic use, and relates to comfort. The term "comfort" refers to the feeling of ease or well-being. Especially, a nutraceutical composition may be used for promoting, maintaining and/or improving comfort or for alleviating and/or preventing a discomfort. A nutraceutical composition may be in the form of a nutritional product, such as, for example, a functional food or a food or dietary supplement.

**"Nutraceutically effective amount"** refers to the amount of a nutraceutical composition, food or dietary supplement or functional food necessary and sufficient for providing a physiological benefit or alleviating a discomfort.

The term **"administration",** or a variant thereof (e.g., **"administering"**), means providing the active agent, herein the herbal composition, alone or as part of a pharmaceutically and/or nutraceutically acceptable composition, to the patient in whom/which the condition, symptom, or disease is to be treated.

The term **"rhizome"** is a botanical term referring to a modified subterranean plant stem that sends out roots and shoots from its nodes.

The term **"subject"** refers to a mammal, preferably a human. According to the present invention, a subject is a mammal, preferably a human. In one embodiment, the subject is a "patient", i.e., a mammal, preferably a human, who/which is awaiting the receipt of, or is receiving medical care or was/is/will be the object of a medical procedure or is monitored for the development of a condition, symptom, disorder or disease.

The term **"human"** refers to a subject of both genders and at any stage of development (*i.e.,* neonate, infant, juvenile, adolescent, adult).

The terms **"treat", "treating"** or **"treatment",** as used herein, refer to a therapeutic treatment, to a prophylactic (or preventive) treatment, or to both a therapeutic treatment and a prophylactic (or preventive) treatment, wherein the object is to prevent, reduce, alleviate, and/or slow down (lessen) one or more of the symptoms of a condition, disorder or disease, in a subject in need thereof.

### DETAILED DESCRIPTION

According to a first aspect, the invention relates to a herbal composition comprising: a first composition comprising an extract of *Alpinia galanga,* and a second composition comprising an extract of *Phyllanthus emblica.*

Unless otherwise specified, the extracts herein described can be selected from whole plant, fruit, bark, leaf, flower, flowering tops, root and/or rhizhome extracts. Preferably, the extracts may be selected from fruit, flowering tops, root and/or rhizhome extracts.

The first composition comprises or consists of an extract of *Alpinia galanga.* In one embodiment, the first composition consists of an extract of *Alpinia galanga.*

*Alpinia galanga,* also known as the greater galangal, lengkuas or blue ginger, is an aromatic herbaceous edible plant belonging to the Zingiberaceae family. It is of note that *Alpinia galanga* designates a different species from *A. officinarum* that is known as lesser galangal.

*Alpinia galanga* is native from South-east Asia. The rhizome of *A. galanga* is a tuberous rhizome, reddish on the surface, that presents characteristic circular rings, with odorous rings and pungent taste.

The phytochemical analysis of *A. galanga* has shown that it is a plant rich in secondary metabolites comprising high levels of essential oil, rich in cineole. Further *A. galanga* constituents that have been identified comprise flavonoids, shogaols, neolignans and phenylpropanoids, of which the main studied is acetoxychavicol acetate (AAC). Recent articles have highlighted the anti-tumor properties of AAC in different animal models.

To the Applicant's knowledge, *A. galanga* has been reported for its potential pharmacological use against digestive and rheumatological disorders as well as HIV infections.

According to a preferred embodiment, the first composition consists of an extract of *Alpinia galanga* rhizomes.

The extract of *A. galanga,* preferably of *A. galanga* rhizomes, may be obtained by any extraction method known in the art. According to an exemplary embodiment, *A. galanga,* preferably *A. galanga* rhizomes, may be crushed, followed by a solid/liquid extraction with a solvent, providing an *A. galanga* extract. Typically, the *A. galanga* extract comprises acetoxychavicol acetate. The operation may be repeated at least twice in order to ensure the quantitative extraction of *A. galanga* metabolites. In one embodiment, the solvent is ethanol, providing an ethanolic *A. galanga* extract. In one embodiment, the solvent is methanol, providing an methanolic *A. galanga* extract. In one embodiment, the solvent is a mixture of ethanol with water, providing a hydroalcoholic *A. galanga* extract. According to one embodiment, the solvent is a mixture of ethanol with water in an ethanol:water volume ratio ranging from 40:60 to 96:4, from 50:50 to 90:10, from 60:40 to 85:15, or from 70:30 to 80:20. According to one embodiment, the solvent is 100% water.

The obtained extract can be concentrated by removing the solvent, such as for example by subjecting the extract to reduced pressure and optionally heat.

According to a specific embodiment, the first composition comprises or consists of an ethanolic extract of *Alpinia galanga* rhizomes.

The second composition comprises or consists of an extract of *Phyllanthus emblica.*

*Phyllanthus emblica* was previously indexed as *Emblica officinalis,* is also known as embli, emblic myrobalan, myrobalan, Indian gooseberry, Malacca tree, or amla, and is a deciduous tree of the family Phyllanthaceae. *Phyllanthus emblica* contains high amounts of ascorbic acid and has a bitter taste that is attributed to a high density of ellagitannins, such as emblicanin A, emblicanin B, punigluconin, and pedunculagin. *Phyllanthus emblica* also contains polyphenols, such as punicafolin and phyllanemblinin A, phyllanemblin, ellagic acid, gallic acid and flavonoids, such as kaempferol. Due to its astringent taste, *Phyllanthus emblica* is commonly used in the Indian medicine.

In one typical embodiment, the second composition consists of an extract of *Phyllanthus emblica,* preferably consists of an extract of *Phyllanthus emblica* fruits.

The *Phyllanthus emblica* may be in the form of a mix know in the art as Triphala extract mix. The Triphala extract mix is used in the Indian medicine for kidney and liver dysfunctions.

Thus, in one optional embodiment, the second composition comprises or consists of a mixture of *Phyllanthus emblica, Terminalia chebula* and *Terminalia bellirica* extracts. In one embodiment, the second composition consists of a mixture of *Phyllanthus emblica, Terminalia chebula* and *Terminalia bellirica* extracts.

*Terminalia chebula,* commonly known as black- or chebulic myrobalan, is a deciduous tree of the Combretaceae family, native from South Asia and South-east Asia. The *Terminalia chebula* chemical composition comprises triterpenes, triterpene glycosides, phenolic compounds including ellagic acid, 2,4-chebulyl-β-*D*-glucopyranose, tannins such as chebulinic acid, gallic acid, ethyl gallate, punicalagin, terflavin A, terchebin, luteolin, and tannic acid. The dried *Terminalia chebula* fruit is also used in Indian medicine as a purported antitussive, cardiotonic, homeostatic, diuretic, and laxative.

*Terminalia bellirica,* also indexed as *Terminalia bellerica,* is also known as baheda, bahera, beleric or bastard myrobalan, and is a deciduous tree of the Combretaceae family. The fruits of *T. bellirica* are reported as a multi-use therapeutic herbal product in the Tibetan medicine and are prescribed as a general health tonic in the traditional Indian medicine. The chemical constituents of *T. bellirica* comprise tannins, ellagic acid, ethylgallate, gallylglucose, and chebulinic acid. The reported *Terminalia bellirica* uses comprise its use as antioxidant, antimicrobial, antidiarrhoeal, anticancer, antidiabetic, antihypertensive and hepatoprotective agent.

Each of the *Phyllanthus emblica, Terminalia chebula* and *Terminalia bellirica* extracts, preferably fruit extracts, may be obtained by any extraction method known in the art or as indicatively presented herein above for the first composition. In one embodiment, each of the *Phyllanthus emblica, Terminalia chebula* and *Terminalia bellirica* extracts, preferably fruit extracts, is an aqueous extract. In one embodiment, the aqueous extract is obtained with a solvent comprising at least 90% water, at least 95% water, at least 99% water, or 100% water.

Optionally, when the second composition comprises or consists of the Triphala extract mixture, the relative weight proportion of *Phyllanthus emblica, Terminalia chebula* and *Terminalia bellirica* extracts, preferably fruit extracts, typically dry fruit extracts, in the second composition is 1:1:1.

According to an optional embodiment, the second composition comprises or consists of an aqueous extract of *Phyllanthus emblica, Terminalia chebula* and *Terminalia bellirica* fruits. The second composition may be obtained by mixing *Phyllanthus emblica, Terminalia chebula* and *Terminalia bellirica,* preferably their fruits, and then extracting them as detailed above. Alternatively, *Phyllanthus emblica, Terminalia chebula* and *Terminalia bellirica,* preferably their fruits, are extracted separately as detailed above and then the obtained extracts, preferably the obtained aqueous extracts, are mixed together to form Triphala or the second composition according to the present invention.

Optionally, the herbal composition further comprises a third composition comprising or consisting of *Filipendula ulmaria* or an extract thereof. In the context of the present specification, the third composition may be a dried and/or milled *Filipendula ulmaria* plant material composition or an extract thereof. Typically, the *Filipendula ulmaria* plant material comprises or consists of *Filipendula ulmaria* aerial parts, preferably the flowering tops. According to preferred embodiments, the third composition comprises or consists of dried and/or milled *Filipendula ulmaria* plant material, typically aerial parts, preferably the flowering tops. According to some alternative embodiments, the third composition comprises or consists of an extract of *Filipendula ulmaria* plant material, typically an extract of its aerial parts, preferably an extract of its flowering tops.

According to a preferred embodiment, the third composition consists of *F. ulmaria* aerial parts, typically dried and/or milled aerial parts.

According to a preferred embodiment, the third composition consists of *F. ulmaria* flowering tops, typically dried and/or milled flowering tops.

*Filipendula ulmaria,* also known as meadowsweet, meadow wort or queen of the meadow, is an herbaceous plant of the Rosaceae family. Traditional uses of *F. ulmaria* in Europe include the treatment of rheumatism, gout, infections, and fever. Phytochemical constituents of *F. ulmaria* comprise salicylic acid, flavone glycosides, essential oils, and tannins.

The extract of *F. ulmaria,* typically of *F. ulmaria* aerial parts, preferably of *F. ulmaria* flowering tops, may be obtained by any extraction method known in the art. According to an exemplary embodiment, *F. ulmaria,* typically of *F. ulmaria* aerial parts, preferably of *F. ulmaria* flowering tops, may be crushed, followed by a solid/liquid extraction with a solvent, providing an *F. ulmaria* extract. The operation may be repeated at least twice in order to ensure the quantitative extraction of *F. ulmaria* metabolites. In one embodiment, the solvent is ethanol, providing an ethanolic *F. ulmaria* extract. In one embodiment, the solvent is methanol, providing an methanolic *F. ulmaria* extract. In one embodiment, the solvent is a mixture of ethanol with water, providing a hydroalcoholic *F. ulmaria* extract. According to one embodiment, the solvent is a mixture of ethanol with water in an ethanol:water volume ratio ranging from 40:60 to 96:4, from 50:50 to 90:10, from 60:40 to 85:15, or from 70:30 to 80:20. According to one embodiment, the solvent is 100% water.

Advantageously, the first, the second and optionally the third compositions are fermented compositions with at least one yeast. In one embodiment, the first and/or the second compositions are fermented compositions with at least one yeast.

In one first variant, the first and the second compositions are fermented compositions with said at least one yeast. In one second variant, the first and the third compositions are fermented compositions with said at least one yeast. In one third variant, the second and the third compositions are fermented compositions with said at least one yeast. Preferably, the first and the second compositions are fermented compositions with said at least one yeast. In one embodiment, the first and/or the second compositions are fermented compositions with at least one yeast and the third composition is non-fermented.

In one typical embodiment, the herbal composition is a fermented herbal composition. It should be understood that, in the context of the present specification, the term "composition" can refer to the embodiments of the invention wherein the (first, second and/or third) composition is fermented as well as to the embodiments wherein said composition is not fermented. Likewise, in the context of the present specification, the term "herbal composition" can refer to the embodiments of the invention wherein the herbal composition is fermented as well as to the embodiments wherein the herbal composition is not fermented.

In one embodiment, the first, the second and optionally the third compositions are fermented with the same at least one yeast. In another embodiment, each of the first, the second and the third extracts is fermented with a different yeast.

According to a preferred embodiment, the at least one yeast is a yeast of the Saccharomycetaceae family. Indicative Saccharomycetaceae family genera can be selected from the group consisting of *Brettanomyces, Candida, Debaromyces, Kluyveromyces, Pichia, Torulaspora, Zygosaccharomyces* and *Saccharomyces.*

Preferably, the at least one yeast is at least one *Saccharomyces* yeast. The at least one *Saccharomyces* genus yeast may be selected from the group consisting of *Saccharomyces cerevisiae, S. cerevisiae* var *boulardii, S. arboricolus, S. bayanus, S. coriocanus, S. chevallierun S. eubayanus, S. florentinus, S. kudriavzevii, S. mikatae, S. paradoxus, S. pastorianus,* and *S. uvarum.*

Even more preferably, the at least one yeast is *Saccharomyces cerevisiae. Saccharomyces cerevisiae,* also known as brewers' yeast, is commonly used in winemaking, baking, and brewing. *S. cerevisiae* is also reported as a food complement. The oral use of *Saccharomyces cerevisiae* is generally recognized as safe.

According to a first variant, the first, the second and optionally the third compositions are fermented separately with the at least one yeast prior to be mixed in order to form the herbal composition according to the invention. According to a second and preferred variant, the first, the second and optionally the third compositions are mixed together prior to being (jointly) fermented with the at least one yeast.

The herbal composition according to the invention may be solid, liquid or in the form of a paste. In one embodiment, the herbal composition is liquid or in the form of a paste, preferably the herbal composition is liquid. It should be understood that the state of the herbal composition is described at atmospheric pressure and at room temperature (25°C).

In one embodiment, the amount of the first and/or the second composition ranges from 5 % to 80 %, from 5 to 750%, or from 25 to 75 % in weight relative to the total weight of the herbal composition. It is understood that the sum of the weight percentages does not exceed 100%.

In one embodiment, the amount of the first composition ranges from 5 % to 60, from 5 to 40%, or from 10 to 30 % in weight relative to the total weight of the herbal composition. In one specific embodiment, the amount of the first composition ranges 20 to 25 % in weight relative to the total weight of the herbal composition.

In one embodiment, the amount of the second composition ranges from 5 % to 80, from 10 to 80%, or from 20 to 75 % in weight relative to the total weight of the herbal composition. In one specific embodiment, the amount of the second composition ranges from 70 to 75 % in weight relative to the total weight of the herbal composition.

In one embodiment, the amount of the third composition ranges from 0.5 % to 20, from 0.5 to 10%, or from 1 to 3 % in weight relative to the total weight of the herbal composition. In one embodiment, the amount of the third composition ranges from about 2 % to about 3 % in weight relative to the total weight of the herbal composition.

In one embodiment, the herbal composition according the invention comprises or consists of:
- from 5 % to 60 %, from 5 to 40%, or from 10 to 30 % in weight relative to the total weight of the herbal composition of the first composition comprising or consisting of *Alpinia galanga* extract, preferably *A. galanga* rhizome extract, even more preferably *A. galanga* rhizome ethanolic extract, and
- from 5 % to 80 %, from 10 to 80%, or from 20 to 75 % in weight relative to the total weight of the herbal composition of the second composition comprising or consisting of a *Phyllanthus emblica* extract, preferably fruit extract, even more preferably *Phyllanthus emblica* fruit aqueous extract, and
- optionally from 0.5 % to 20 %, from 0.5 to 10%, or from 1 to 3 % in weight relative to the total weight of the herbal composition of the third composition comprising or consisting of *Filipendula ulmaria,* preferably a *Filipendula ulmaria* aerial parts , even more preferably *Filipendula ulmaria* flowering tops, or an extract thereof.

In one embodiment, the herbal composition or the fermented herbal composition comprises from 1 g/L to 70 g/L, from 1 g/L to 20 g/L, from 5 g/L to 15 g/L, from 1 g/L to 10 g/L, or about 10 g/L of the first composition comprising or consisting of the extract of *Alpinia galanga,* preferably *A. galanga* rhizome extract, even more preferably *A. galanga* rhizome ethanolic extract. In one embodiment, the herbal composition or the fermented herbal composition comprises from 5 g/L to 10 g/L, from 5 g/L to 7 g/L, or about 6.67 g/L of the first composition comprising or consisting of the extract of *Alpinia galanga,* preferably *A. galanga* rhizome extract, even more preferably *A. galanga* rhizome ethanolic extract.

In one embodiment, the herbal composition or the fermented herbal composition comprises from 10 g/L to 70 g/L, from 20 g/L to 60 g/L, from 30 g/L to 50 g/L, or about 45 g/L of the first composition comprising or consisting of the extract of *Alpinia galanga,* preferably *A. galanga* rhizome extract, even more preferably *A. galanga* rhizome ethanolic extract.

In one embodiment, the herbal composition or the fermented herbal composition comprises from 1 g/L to 70 g/L, from 5 g/L to 50 g/L, from 10 g/L to 30 g/L, or about 20 g/L of the second composition comprising or consisting a *Phyllanthus emblica* extract, preferably a *Phyllanthus emblica* fruit extract, even more preferably a *Phyllanthus emblica* fruit aqueous extract.

In one embodiment, the herbal composition or the fermented herbal composition comprises from 10 g/L to 70 g/L, from 20 g/L to 60 g/L, from 20 g/L to 50 g/L of the second composition comprising or consisting a *Phyllanthus emblica* extract, preferably a *Phyllanthus emblica* fruit extract, even more preferably a *Phyllanthus emblica* fruit aqueous extract.

In one embodiment, the herbal composition or the fermented herbal composition comprises from 10 g/L to 70 g/L, from 20 g/L to 60 g/L, from 30 g/L to 50 g/L, or about 45 g/L of the second composition comprising or consisting of a mixture of *Phyllanthus emblica, Terminalia chebula* and *Terminalia bellirica* extracts, preferably fruit extracts, even more preferably fruit aqueous extracts.

In one embodiment, the herbal composition or the fermented herbal composition comprises from 0.1 g/L to 5 g/L, from 0.5 g/L to 1.5 g/L, from 0.5 g/L to 1.0 g/L, or about 0.67 g/L of the third composition comprising or consisting of *Filipendula ulmaria,* preferably *Filipendula ulmaria* aerial parts, even more preferably a *Filipendula ulmaria* flowering tops, or an extract thereof.

In one embodiment, the herbal composition according the invention comprises or consists of:
- from 1 g/L to 70 g/L, from 1 g/L to 50 g/L or from 1 g/L to 20 g/L of the first composition of *Alpinia galanga* extract, preferably *A. galanga* rhizome extract, even more preferably *A. galanga* rhizome ethanolic extract, and
- from 1 g/L to 70 g/L of the second composition comprising a *Phyllanthus emblica* extract, preferably fruit extract, even more preferably fruit aqueous extract.

In one embodiment, the herbal composition according to the invention comprises or consists of:
- from 1 g/L to 70 g/L, from 1 g/L to 50 g/L or from 1 g/L to 20 g/L of the first composition of *Alpinia galanga* extract, preferably *A. galanga* rhizome extract, even more preferably *A. galanga* rhizome ethanolic extract, and
- from 1 g/L to 70 g/L of the second composition consisting of a mixture of *Phyllanthus emblica, Terminalia chebula* and *Terminalia bellirica* extracts, preferably fruit extracts, even more preferably fruit aqueous extracts.

In one embodiment, the herbal composition according the invention comprises or consists of:
- from 5 g/L to 10 g/L, from 5 g/L to 7 g/L, or about 6.67 g/L of the first composition of *Alpinia galanga* extract, preferably *A. galanga* rhizome extract, even more preferably *A. galanga* rhizome ethanolic extract,
- from 5 g/L to 50 g/L, from 10 g/L to 30 g/L or abour 20 g/L of the second composition comprising a *Phyllanthus emblica* extract, preferably fruit extract, even more preferably fruit aqueous extract, and
- from 0.1 g/L to 5 g/L, from 0.5 g/L to 1.5 g/L, from 0.5 g/L to 1.0 g/L, or about 0.67 g/L of the third composition comprising or consisting *Filipendula ulmaria,* preferably *Filipendula ulmaria* aerial parts, even more preferably a *Filipendula ulmaria* flowering tops, or an extract thereof.

In one specific embodiment, the herbal composition according the invention comprises or consists of:
- about 6.67 g/L of the first composition of *Alpinia galanga* extract, preferably A. *galanga* rhizome extract, even more preferably *A. galanga* rhizome ethanolic extract,
- about 20 g/L of the second composition comprising a *Phyllanthus emblica* extract, preferably fruit extract, even more preferably fruit aqueous extract, and
- about 0.67 g/L of the third composition comprising or consisting *Filipendula ulmaria,* preferably *Filipendula ulmaria* aerial parts, even more preferably *Filipendula ulmaria* flowering tops.

The invention further relates to a pharmaceutical composition that comprises the herbal composition according to the invention. Preferably, the pharmaceutical composition comprises the herbal composition in association with at least one pharmaceutically acceptable excipient.

In one embodiment, the at least one pharmaceutically acceptable excipient is selected from protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The pharmaceutical composition may also contain conventional pharmaceutical additives and adjuvants, excipients and diluents, including, but not limited to, water, gelatin of any origin, vegetable gums, lignin sulfonate, talc, sugars, starch, gum arabic, vegetable oils, polyalkylene glycols, preservatives such as sodium benzoate and/or potassium sorbate, stabilizers, emulsifying agents, buffers, lubricants, colorants, wetting agents, fillers, and flavoring agents.

The invention further relates to a nutraceutical composition that comprises the herbal composition according to the invention. Preferably, the nutraceutical composition comprises the herbal composition in association with at least one nutraceutically acceptable excipient. Examples of nutritionally acceptable excipients include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroetheral fatty acid esters, hydroxymethyl-cellulose, polyvinylpyrrolidone, flavoring agents, preservatives such as sodium benzoate and/or potassium sorbate.

In one embodiment, the pharmaceutical or nutraceutical composition is an oral pharmaceutical or nutraceutical composition. In other terms, the pharmaceutical or the nutraceutical composition is suitable for oral administration or ingestion.

In one embodiment, the pharmaceutical or the nutraceutical composition is a liquid composition. The liquid pharmaceutical or nutraceutical composition may be formulated in the form at least one vial. The at least one vial may contain 10 mL, 20 mL or 30 mL of the pharmaceutical or the nutraceutical composition comprising the herbal composition as defined above.

Typically, the liquid pharmaceutical or nutraceutical composition may be formulated in the form of a packaging comprising at least one, at least five, at least ten, or at least twenty vials as described above.

It should be understood that a kit-of-parts is also in the scope of the present invention. For instance, such kit-of-parts may comprise a first part comprising the first composition, optionally in association with at least one pharmaceutically or nutraceutically acceptable excipient, a second part comprising the second composition, optionally in association with at least one pharmaceutically or nutraceutically acceptable excipient, and optionally a third part comprising the third composition, optionally in association with at least one pharmaceutically or nutraceutically acceptable excipient. In one embodiment, the kit-of-parts may comprise a first part comprising the first and the second compositions and a second part comprising the third composition.

In one embodiment, the kit-of-parts may comprise a first part comprising the third and the second compositions and a second part comprising the first composition. In one embodiment, the kit-of-parts may comprise a first part comprising the first and the third compositions and a second part comprising the second composition.

The invention further relates to the non-therapeutic use of a composition or a nutraceutical composition according to the invention in the management of immune-system well-being. In some embodiments, the invention relates to the use of a nutraceutical composition according to the invention for alleviating respiratory symptoms.

According to a second aspect, the invention relates to a process for preparing a herbal composition according to the invention.

According to a first embodiment, the process comprises the steps of:
i. supplying a first composition comprising or consisting of an extract of *Alpinia galanga,* preferably an extract of *Alpinia galanga* rhizomes
ii. supplying second composition comprising an extract of *Phyllanthus emblica,* preferably an extract of *Phyllanthus emblica* fruits,and optionally further comprising *Terminalia chebula* and *Terminalia bellirica* fruit extracts,
iii. optionally supplying a third composition comprising or consisting *Filipendula ulmaria,* preferably *Filipendula ulmaria* aerial parts, even more preferably *Filipendula ulmaria* flowering tops, or an extract thereof,
iv. mixing the first with the second compositions and optionally with the third composition, leading to a herbal composition.

According to a typical embodiment, the process further comprises the steps of:
v. fermenting the herbal composition with at least one yeast, preferably at least one Saccharomycetaceae strain, even more preferably *Saccharomyces cerevisiae,* leading to a fermented herbal composition,
vi. optionally condensing the fermented herbal composition, leading to a condensed herbal composition, and
vii. optionally mixing the fermented herbal composition of step (v) or the condensed herbal composition of step (vi) with at least one pharmaceutically and/or nutraceutically acceptable excipient.

In one embodiment, the third composition described in step (iii) is mixed with the fermented herbal composition of step (v).

According to one embodiment, the process comprises the steps of:
i'. supplying a first composition comprising or consisting of an extract of *Alpinia galanga,*
ii'. supplying second composition comprising an extract of *Phyllanthus emblica,* and optionally further comprising *Terminalia chebula* and *Terminalia bellirica* extracts,
iii'. optionally supplying a third composition comprising or consisting *Filipendula ulmaria,* preferably *Filipendula ulmaria* aerial parts, even more preferably *Filipendula ulmaria* flowering tops, or an extract thereof;
iv'. fermenting each of the first, the second and optionally the third compositions with at least one yeast, preferably at least one Saccharomycetaceae strain, even more preferably *Saccharomyces cerevisiae,* leading to a first fermented composition, a second fermented composition and optionally a third fermented composition;
v'. mixing the first, the second, and optionally the third, fermented compositions, leading to the fermented herbal composition.

In one embodiment, the third composition is not fermented and is mixed with the first and the second fermented compositions in step (v').

Supplying the first, the second, and optionally the third, compositions according to steps (i) to (iii) or steps (i') to (iii') may be carried out as described in the description of the invention's composition. Likewise, the amounts of said extracts may be as described in the description of the invention's composition.

The mixing step (iv) or (v') may be carried out by any mixing means known in the art such as for example by stirring, beating or blending the first, the second, and optionally the third, extracts or the first, the second, and optionally the third, fermented extracts.

In one embodiment, the obtained herbal composition is a powder, a paste, a solution or a suspension. Typically, the obtained herbal composition is a paste or a suspension. Without willing to be bound by a theory, fermenting the herbal composition or the first, the second, and optionally the third, extracts leads to an enhancement of the aqueous solubility of the herbal composition due to the hydrolysis of the tannins comprised therein. In one embodiment, the herbal composition according to the invention presents an aqueous solubility of more than 5 mg/mL, of more than 8 mg/mL of more than 10 mg/mL, or of more than 15 mg/mL at 25°C.

The fermenting step (v) or (iv') may optionally comprise a first step of mixing the herbal composition or each of the first, the second, and optionally the third compositions with an aqueous composition in order to obtain a suspension. In one embodiment, the aqueous composition is 100% water. In one embodiment, the aqueous composition further comprises glucose. In one embodiment, the aqueous composition comprises water, glucose and an ammonium source such as ammonium nitrate. The fermenting step (v) or (iv') may be carried out by any means known in the art, such as for example in a bioreactor, typically under agitation, preferably at a temperature ranging from 20°C to 30°C. The fermenting step (v) or (iv') may last at least 12 hours, at least 1 day, at least 2 days, at least 3 days, at least 4 days, at least 5 days, at least 6 days, or at least 7 days. The skilled artisan knows how to adjust the duration of the fermentation step such as for example based on the bioreactor temperature and the presence or absence of glucose and/or of ammonium sources.

Typically, the fermenting step (v) comprises the steps of: a) inoculating the herbal composition of step (iv) or each of the first, the second, and optionally the third compositions with at least one fungal strain, preferably *Saccharomyces cerevisiae,* typically suspended in the hereinabove described aqueous composition and b) fermenting the inoculated mixed extract of step a) for at least 12 hours at a temperature ranging from 20°C to 30°C.

In one embodiment, the obtained fermented herbal composition is a powder, a solution or a suspension. Typically, the obtained fermented herbal composition is a solution or a suspension.

The invention further relates to the herbal composition or the fermented herbal composition that is obtainable or directly obtained by any one of the above process embodiments.

In one embodiment, the herbal composition or the fermented herbal composition that is obtainable or directly obtained by any one of the above process embodiments is the herbal composition or the fermented herbal composition according to the invention as described herein above.

According to a third aspect, the invention relates to a composition according to any one of the above aspects and embodiments, for use as a drug. In one embodiment, the invention relates to the herbal composition according to any one of the above aspects and embodiments for use as a drug. In one embodiment, the invention relates to the fermented herbal composition according to any one of the above aspects and embodiments for use as a drug. In one embodiment, the invention relates to the pharmaceutical composition comprising the herbal composition or the fermented herbal composition according to any one of the above aspects and embodiments, for use as a drug. The invention further relates to the use of the composition according to the invention for the preparation of a drug.

According to one embodiment, the drug is for use in the prevention and/or the treatment of a viral infection in a subject in need thereof. In one embodiment, the drug is for limiting the number of cells infected by the virus, thereby permitting the treatment of the viral infection.

In one embodiment, the viral infection is a pulmonary viral infection by at least one peplomer (spike) bearing virus.

In one embodiment, the peplomer (spike) bearing virus is selected from the group consisting of orthomyxoviruses, paramyxoviruses, rhabdoviruses, filoviruses, coronaviruses, bunyaviruses, arenaviruses, and retroviruses. In one embodiment, the peplomer (spike) bearing virus is selected from the group consisting of influenza virus, cytomegalovirus (Epstein-Barr), papillomavirus (HPV), adenovirus, HIV (Human Immunodeficiency Virus), herpes simplex virus, genital herpes virus and the coronaviruses. In one embodiment, the peplomer (spike) bearing virus is influenza virus. In one embodiment, the peplomer bearing virus is cytomegalovirus (Epstein-Barr). In one embodiment, the peplomer bearing virus is papillomavirus (HPV). In one embodiment, the peplomer bearing virus is adenovirus. In one embodiment, the peplomer bearing virus is HIV. In one embodiment, the peplomer bearing virus is herpes simplex virus. In one embodiment, the peplomer bearing virus is genital herpes virus.

In one typical embodiment, the peplomer bearing virus is selected from coronaviruses.

In one embodiment, the peplomer bearing virus is a coronavirus. In one embodiment, the peplomer bearing virus is SARS-CoV-2 virus. SARS-CoV-2 is known for causing the COVID-19 disease and associated disorders.

Thus, treating SARS-CoV-2 virus also refers to treating COVID-19 disease and associated disorders. As used herein, COVID-19 disease and associated disorders denote diseases or disorders associated with infection from SARS-CoV-2 virus and strains/variants thereof, including fatigue, shortness of breath or difficulty in breathing, cough, joint pain, chest pain, memory, concentration or sleep problems, muscle pain, headache, fast or pounding heartbeat, loss of smell or taste, depression or anxiety, fever, dizziness, blood clotting, myocardial infarction, lung damage, severe acute respiratory syndrome (SARS), renal failure, inflammation, strokes, seizures, Guillain-Barre syndrome and such other disorders as known to or appreciated by persons skilled in the art.

The invention further relates to a method for treating a viral infection or an associated disease as defined above, said method comprising the administration, preferably the oral administration, of a therapeutically effective amount of a composition according to the invention to a subject in need thereof. In one embodiment, the composition is the pharmaceutical composition according to the invention.

In one embodiment, the subject is a male or female human subject. In one embodiment, the subject is a subject not suffering from other pathologies other than the SARS-CoV-2 infection and/or the COVID-19 disorders. In one embodiment, the subject suffers from COVID-19 comorbidity pathologies such as asthma, metabolic syndrome, or immune system overreaction. In one embodiment, the subject is an elderly subject, that is an aging subject past the stage of maturity. In one embodiment, an elderly human subject is of at least 45, at least 50 or at least 55 years old.

The posology may vary within a wide range depending on the therapeutic and/or nutraceutical indication and the route of administration, and also the general health, age, sex, and body weight of the individual. The skilled artisan will be able to determine appropriate dosages depending on these and other factors.

In one embodiment, the composition or the pharmaceutical composition is orally administered to the subject at least once per week, at least two or three times per week or at least once daily. For instance, the subject may ingest the content of a 10 mL, of a 15 mL or of a 20 mL vial comprising the composition or the pharmaceutical composition as described above at least once a day.

The herbal composition or the pharmaceutical composition may be administered sequentially to or simultaneously with at least one second pharmaceutical composition that is suitable for preventing and/or treating a viral disease.

In one embodiment the at least one second pharmaceutical composition comprises an antiviral agent selected from the group consisting of a virus entry inhibitors, virus early transcription event inhibitors, virus RNA polymerase inhibitors such as remdesivir, virus protease inhibitors such as lopinavir or ritonavir, virus terminase inhibitors, virus maturation inhibitors, inhibitors of another target in the virus life cycle such as , hydroxychloroquine or pharmaceutically acceptable salts thereof and a vaccine. In one embodiment the at least one second pharmaceutical composition comprises an antiviral agent selected from the group consisting of remdesivir, lopinavir, ritonavir and hydroxychloroquine.

These additional agents may be combined with the antiviral compound or antiviral composition or pharmaceutical compositions of this invention to create a single pharmaceutical dosage form. Alternatively, these additional agents may be separately administered to the patient as part of a multiple dosage form, for example, using a kit. Such additional agents may be administered to the patient prior to, concurrently with, or following the administration of a pharmaceutical composition of the invention.

### EXAMPLES

The present invention is further illustrated by the following examples.

### Example 1: Preparation of a herbal composition according to the invention

3.5 kg of *Alpinia Galanga* roots from the company ALP ERBO (43 boulevard des tilleuls F04100 Manosque) were crushed, then introduced into a solid /liquid extractor in the presence of 8 liters of 96% ethanol (Carbo ERBA CAS number: 64-17-5).

After 1 hour at 60°C, the mixture was filtered and the alcoholic solution was concentrated under reduced pressure, yielding 230 grams of a pasty fragrant extract.

The extraction operation was repeated with the same plant residue wherein 5 liters of ethanol were added. After filtration and concentration, 270 grams of a pasty extract were obtained. The overall extraction yield is 14% by mass.

The *Phyllanthus emblica* extract was supplied in the form of a Triphala extract mixture, that was commercially available from the company L'HORIZON NATURE (88, rue Paul Bert - 69400 Villefranche sur Saône).

100 mg of the first composition (*A. galanga* extract), and 450 mg of the second composition (*Phyllanthus emblica,* Triphala mix) were mixed, yielding the herbal composition.

### Example 2: Pre-clinical toxicity assessment

The subacute toxicity of a herbal composition comprising *Alpinia galanga* and *Phyllanthus emblica* was assessed at 300 mg / kg *in vivo.*

Two groups of mice (8 weeks old, male and female) were processed, 6-8 mice in each group weighing between 22 and 42 g each. The oral administration of the herbal composition was carried out by gavaging following OECD guidelines No. 407.
- Group 1: served as a control group receiving normal saline solution for 28 days,
- Group 2: received the herbal composition daily at a dose of 300 mg/kg per body weight for 28 days.

The monitoring of toxicological parameters indicative of any sub-acute toxicity was carried out on the batches studied during the 4 weeks of the experimental protocol.

Animals were observed individually at least once during the first 30 minutes and regularly during the first 24 hours after treatment. Special care should be taken during the first 4 hours and daily for 28 days after administration of the substance.

No mortality or behavioral alteration was reported for the two products tested during the 28 days of subacute administration.

The macroscopic inspection of the internal organs after the study revealed no alteration. The renal and hepatic histological alterations were reported at the study group.

No significant difference in the biochemical parameters relating to hepatic lesions (ASAT, ALAT, etc.), renal dysfunction (urea, creatinine, etc.) was demonstrated after the administration of the dose of 300 mg/kg.

### Example 3: Acetoxychavicol acetate (AAC) antiviral effect

The purpose of this example is to characterize the anti- SARS-CoV-2 activity of the AAC compound in a model of pre-incubation compound / SARS-CoV-2 prior to Vero E6 cells infection. All study procedures were performed according to the approved written study protocol and standard operating procedures (SOPs). All experimental protocols involving live SARS-CoV-2 followed the approved standard operating procedures of the Biosafety Level 3 facility.

### Materials and methods

SARS-CoV-2 was isolated from a patient with laboratory-confirmed COVID-19 in Toulouse. The viral isolate was amplified by one additional passage in Vero E6 cells to make working stocks of the virus.

Vero E6 Cells (ATCC) were cultured in Dulbecco's modified Eagle's medium (DMEM) supplemented with 10% v/v fetal bovine serum (ATCC), 1% v/v penicillin-streptomycin supplemented with 1% v/v sodium pyruvate.

Vero cells were seeded at 1 x 10⁵ cells per well in 12-well tissue culture plates. At 100% confluence (2 days post-seeding), the cells were washed twice with PBS and six serial dilutions of the virus (1/10 each time) were added to the cells. Following infection with 0.3 mL per well of each dilution, plates were incubated at 37°C for 1h, and the cells were washed with PBS before the addition of 2% w/v agar containing 1 µg ml-5 tosyl phenylalanyl chloromethyl ketone-trypsin (Sigma-Aldrich) to the cell surface.

Plates were left at room temperature for 20-30 min to allow for the overlay to set, and were then incubated at 37°C for 72h. Cells were fixed with 4% v/v paraformaldehyde before both the fixative and agar will be removed and the cells stained with 0.1% w/v Crystal Violet (Fisher) in 20% v/v ethanol. Virus titer was determined as plaque forming units (p.f.u.) per mL.

Vero E6 cells were seeded in 96-well clusters at a density of 5,000 cells/well two days before infection.

### Treatment

4 concentrations of the AAC compound (1 µM, 10 µM, 100 µM ans 1 mM) were mixed with 300 pfu of SARS-CoV-2 (final volume 200 µL). This mixture was incubated for 3 hours at 37°C in a humidified atmosphere with 5% CO₂. After incubation (Incubator Thermo^{®} 381), 100 µL of each dilution were added in 6 wells of a cell plate containing a semi-confluent Vero E6 cells monolayer.

Control cells were infected with Covid-19 at MOI (Multiplicity of infection) 0.01. Remdesivir (25 µM) was used as a positive control.

After 55 hours of incubation, the plates were inspected by an inverted optical microscope. Viable cells were quantified with CellTiter-Glo 2.0 luminescent cell viability assay (Promega).

### Results

Treatment with Remdesivir at 25µM, a compound of reference in the treatment of SARS-CoV-2 infection, fully protected the cells from SARS-CoV-2 infection (97% viability in comparison with non-infected cells) thereby validating the present experimental protocol.

AAC, that is typically present in the first composition according to the invention, induced a cytotoxicity on Vero E6 cells at concentrations of 100 µM and 1 mM and no antiviral effect could be observed.

However, at 1 µM and 10 µM, cell viability was respectively 59 and 66% in comparison with non-infected cells. The treatment with AAC at 1 µM and 10 µM, showed a partial protection from SARS-CoV-2 infection after 55 hours of incubation.

### Example 4: Herbal composition's antiviral effect

The purpose of this example is to characterize the anti- SARS-CoV-2 activity of the herbal composition of the invention in a model of pre-incubation compound / Sars-CoV-2 prior to Vero E6 cells infection.

Using the material and methods according to Example 3, the following treatment was applied to the Vero E6 cells prior to their infection with SARS-CoV-2.

### Treatment

Two-fold serial dilutions of the herbal composition (1mg/µL, 500 µg/µL, 250µg/µL ... up to 2µg/µL) solubilized in DMSO were mixed with 250 pfu of SARS-CoV-2 (final volume 150 µL). This mixture was incubated for 3 hours at 37°C in a humidified atmosphere with 5% CO₂. After incubation, 100 µL of each dilution was added to wells of a cell plate containing a semi-confluent Vero E6 cells monolayer. Control wells with the vehicle (different concentrations of DMSO) was included.

After 3 days of incubation, the plate was inspected by an inverted optical microscope. Viable cells were quantified with CellTiter-Glo 2.0 luminescent cell viability assay.

### Results

DMSO, the vehicle used to dissolve the composition, showed an important cytotoxic effect of the vehicle for all concentrations.

Despite the negative DMSO effect, co-incubation of the virus with 4 to 8 µg/µL of the composition resulted in a 40 % restauration of cell viability (compared to the 11 % cell viability measured when the virus was not treated, i.e. untreated cells infected by MOI 0.01). Furthermore, the co-incubation of the virus with 16 to 62 µg/µL of the composition resulted in a 60 % restauration of cell viability (compared to the 11 % cell viability measured when the virus was not treated, i.e. untreated cells infected by MOI 0.01)

Thus, despite the negative DMSO effect, co-incubation of the products with the virus for 3h prior to infection drastically reduced the infectivity of the virus.

### Example 5: Preparation of a fermented herbal composition according to the invention

In order to enhance the aqueous solubility of the herbal composition of the invention, and thereby in order to reduce the necessary amount of solubilizing vehicles such as DMSO, the following fermentation treatment was carried out. The present example further assesses the biotransformation of *A. galanga* and *P. emblica* chemical constituents upon fermentation.

The hereinafter described fermentation processes were carried out with and without the present of glucose. The evolution of the fermentation process was carried out by HPLC coupled to a PDA/DEDL detector at days D0, D1, D5 and D8 of the fermentation.

### A. galanga fermentation

1.5 g of *A. galanga* rhizomes extract were solubilized in 10 mL of ethanol, then diluted with 300 mL of water wherein 10g of brewer's yeast (*S. cerevisiae*) were dispersed.

The same fermentation was carried out by further adding 10 g of glucose.

At D0, the solution was cloudy, slightly colored, the presence of a peak is observed at a retention time of 26.40 minutes which corresponds to acetoxychavicol acetate (AAC).

At D5, the formation of two new products is observed, respectively at retention times of 19.1 and 19.6 minutes and there is still AAC.

At D7, no further modifications were observed implying that the fermentation is complete.

The addition of glucose did not influence the metabolism of *A. galanga* extract by brewer's yeast. The appearance of two compounds with retention times of 19.1, 19.6 and implies the hydrolysis of compounds, probably tannins.

### P. emblica fermentation

1.5 g of *P. emblica* rhizomes extract were solubilized in 300 mL of water wherein 10g of brewer's yeast (*S. cerevisiae*) were dispersed.

The same fermentation was carried out by further adding 10g of glucose.

The HPLC analysis at D0 revealed the markers at retention time 8.3 minutes (Gallic acid); 9.6 minutes; 12.2 minutes and 13 minutes

From D1 to D5, in the presence and absence of glucose, a disappearance of the markers is observed at 9.6 and 12.2 and 13 minutes.

On D8, in the presence of glucose, the reaction no longer evolves. In the absence of glucose, a strong degradation of gallic acid is observed.

Thus, it can be concluded that the fermentation with brewer's yeast leads to the hydrolysis of gallic tannins present in the *P. emblica* extract.

### Joint A. galanga and P. emblica fermentation

When incubating the mixture of the two extracts under the conditions described above the result is a combination of the results of the observations made on the incubations of the two extracts separately.

## Claims

1. A herbal composition comprising a first composition comprising an extract of *Alpinia galanga* and a second composition comprising an extract of *Phyllanthus emblica,* wherein the first and/or the second compositions are fermented compositions with at least one yeast.

2. The composition according to claim **1,** wherein the first and the second compositions are fermented compositions that have been previously mixed and jointly fermented with at least one fungal strain.

3. The composition according to claim **1** or claim **2,** wherein the at least one fungal strain is *Saccharomyces cerevisiae.*

4. The herbal composition according to any one of claims **1** to **3,** wherein the first composition consists of an *Alpinia galanga* rhizome extract and/or wherein the second composition consists of an extract of *Phyllanthus emblica* fruit extract.

5. The herbal composition according to any one of claims **1** to **4,** further comprising a third composition comprising *Filipendula ulmaria* or an extract thereof.

6. The composition according to any one of claims **1** to **5,** wherein the amount of the first and/or the second composition ranges from 5 % to 60 % in weight relative to the total weight of the composition.

7. The composition according to any one of claims **1** to **6,** said composition presenting an aqueous solubility of more than 5 mg/mL at 25°C.

8. A pharmaceutical composition comprising the composition according to any one of claims **1** to **7** and at least one pharmaceutically acceptable excipient.

9. A nutraceutical composition comprising the composition according to any one of claims **1** to **7** and at least one nutraceutically acceptable excipient.

10. The composition according to any one of claims **1** to **7** or the pharmaceutical composition according to claim **8,** for use as a drug.

11. The composition for use according to any one of claims **1** to **7** or the pharmaceutical composition according to claim **8,** for use in the prevention and/or the treatment of a viral infection in a subject in need thereof.

12. The composition for use according to claim **11,** wherein the viral infection is a pulmonary viral infection by at least one peplomer (spike) bearing virus selected from the group consisting of orthomyxoviruses, paramyxoviruses, rhabdoviruses, filoviruses, coronaviruses, bunyaviruses, arenaviruses, and retroviruses, preferably from coronaviruses.

13. The composition for use according to claim **11** or claim **12,** wherein the viral infection is an infection by the virus SARS-CoV-2.

14. The composition for use according to any one of claims **10** to **13,** wherein the composition is orally administered to the subject at least once a day.

15. A process for preparing a composition according to any one of claims **1** to **9,** comprising the steps of:
i. supplying a first composition comprising or consisting of an extract of *Alpinia galanga,* preferably an extract of *Alpinia galanga* rhizomes
ii. supplying second composition comprising an extract of *Phyllanthus emblica,* preferably an extract of *Phyllanthus emblica* fruits,
iii. optionally supplying a third composition comprising or consisting of *Filipendula ulmaria* or an extract thereof,
iv. mixing the first with the second compositions and optionally with the third composition, leading to a herbal composition.
v. fermenting the herbal composition with at least one yeast, preferably at least one Saccharomycetaceae strain, even more preferably *Saccharomyces cerevisiae,* leading to a fermented herbal composition,
vi. optionally condensing the fermented herbal composition, leading to a condensed herbal composition, and
vii. optionally mixing the fermented herbal composition of step (v) or the condensed herbal composition of step (vi) with at least one pharmaceutically and/or nutraceutically acceptable excipient.
